# EUROPEAN PATENT APPLICATION

(11) **EP 3 597 773 A1**
(43) Date of publication of application: **22.01.2020**
(21) Application number: 18767296.9
(22) Date of filing: 14.03.2018
(51) Int. Cl.: C12Q 1/6806, C12N 15/10, G01N 1/40, C01B 33/12

(54) **MICROBIAL CONCENTRATION METHOD USING DIATOMITE AND NUCLEIC ACID EXTRACTION METHOD**

(30) Priority: 15.03.2017 KR 20170032581; 09.03.2018 KR 20180028131
(71) Applicant: University Of Ulsan Foundation For Industry Cooperation, Ulsan 44610 (KR); The Asan Foundation, Seoul 05505 (KR)
(72) Inventor: SHIN, Yong, Seoul 05505 (KR); ZHAO, Fei, Seoul 02444 (KR)
(74) Representative: Roos, Peter
(86) International application number: PCT/KR2018/002994
(87) International publication number: WO 2018/169307

(57) **Abstract**

The present invention relates to a method of concentrating microorganisms and a method of extracting nucleic acids using diatomaceous earth and developed a technology capable of concentrating microorganisms about 4-8 times using the modified diatomaceous earth and directly extracting nucleic acids from the concentrated microorganism using dimethyl adipimidate (DMA). It is expected to significantly reduce external contamination, cost, time and complexity by enabling microbial concentration and nucleic acid extraction techniques in one tube.

## Description

### [Technical Field]

The present invention relates to a method of concentrating microorganisms and a method of extracting nucleic acids using diatomaceous earth, in particular diatomaceous earth modified with a silane compound.

### [Background Art]

Diatoms are unicellular algae that are distributed in various sizes from 2 µm to 2 mm and are representative phytoplankton commonly found on the earth. Because diatoms account for 20-25% of the total primary production of the land and 40% of the marine biomass production, they have played an important role in ecosystem as marine producers for millions of years. Currently, microalgae have high photosynthetic efficiency relative to cell mass and are mainly used for the production of lipids, biofuels, and bioavailable resources by culturing.

In addition, diatomaceous earth is a natural siliceous material which is a main component of diatomaceous cell membrane, and is also known as bio-silica since it is calcined at 450 °C and composed of hydroxyl-rich silica on the surface. The size of the bio-silica is approximately 10-20 µm.

On the other hand, conventional methods for detecting microorganisms do not use the whole solution in at least 1 ml of the patient sample and can detect by extracting using only a portion of the nucleic acid. There is no big problem for large amounts of microorganisms, but for small amounts of microorganisms, it is not possible to accurately detect and control additional infectious diseases and there is a need for studies of concentration methods to utilize all microorganisms as much as possible in at least 1 ml of sample.

Recently, researches are actively underway to utilize diatoms or diatomaceous earth as inorganic materials, bioenvironmental materials, nano-engineering materials or industrial application materials, but research on methods of using the materials for microbial concentration or nucleic acid extraction is insufficient.

### [Disclosure]

### [Technical Problem]

It is an object of the present invention to provide a composition for concentrating microorganisms comprising diatomaceous earth modified with a silane compound as an active ingredient, a method and a kit for concentrating microorganisms using the same; a composition of extracting nucleic acids comprising diatomaceous earth modified with a silane compound as an active ingredient, a method and a kit of extracting nucleic acids using the same; and a composition for concentrating microorganisms and extracting nucleic acids comprising diatomaceous earth modified with a silane compound as an active ingredient, a method and a kit of extracting nucleic acids from the concentrated microorganism simultaneously with concentrating the microorganism using the same.

### [Technical Solution]

In order to achieve the above object, the present invention provides a composition for concentrating microorganisms comprising diatomaceous earth modified with a silane compound as an active ingredient.

The present invention also provides a method of concentrating microorganisms comprising contacting a sample containing microorganisms with diatomaceous earth modified with a silane compound.

The present invention also provides a kit of concentrating microorganisms comprising the composition.

The present invention also provides a composition for extracting nucleic acids comprising diatomaceous earth modified with a silane compound as an active ingredient.

In addition, the present invention provides a method of extracting nucleic acids comprising: (1) reacting by adding diatomaceous earth modified with a silane compound to nucleic acid sample to be extracted; and (2) eluting nucleic acid from a reactant.

In addition, the present invention provides a method of extracting nucleic acids comprising: (1) mixing by adding diatomaceous earth modified with a silane compound to nucleic acid sample to be extracted; (2) reacting by adding any one or more selected from the group consisting of dimethyl adipimidate (DMA), dimethyl pimelimidate (DMP), dimethyl suberimidate (DMS) and dimethyl 3,3'-dithiobispropionimidate (DTBP); and (3) eluting nucleic acid from a reactant.

Furthermore, the present invention provides a kit of extracting nucleic acids comprising the composition.

The present invention also provides a composition for concentrating microorganisms and extracting nucleic acids comprising diatomaceous earth modified with a silane compound as an active ingredient.

In addition, the present invention provides a method of extracting nucleic acids from concentrated microorganism simultaneously with concentrating the microorganism comprising: (1) concentrating microorganism by contacting a sample containing microorganism with diatomaceous earth modified with a silane compound; (2) lysing concentrated microorganism; and (3) eluting nucleic acid from lysate.

In addition, the present invention provides a method of extracting nucleic acids from concentrated microorganism simultaneously with concentrating the microorganism comprising: (1) concentrating microorganism by contacting a sample containing microorganisms with diatomaceous earth modified with a silane compound; (2) lysing concentrated microorganism; (3) reacting by adding any one or more selected from the group consisting dimethyl adipimidate (DMA), dimethyl pimelimidate (DMP), dimethyl suberimidate (DMS) and dimethyl 3,3'-ditihiobispropionimidate (DTBP) to lysate; and (4) eluting nucleic acid from a reactant.

The present invention also provides a kit of concentrating microorganisms and extracting nucleic acids comprising the composition.

The present invention also provides the use of diatomaceous earth modified with a silane compound for microbial concentration.

The present invention also provides the use of diatomaceous earth modified with silane compounds for nucleic acid extraction.

The present invention also provides the use of diatomaceous earth modified with silane compounds for microbial concentration and nucleic acid extraction.

### [Advantageous Effects]

The present invention relates to a method of concentrating microorganisms and extracting nucleic acid using diatomaceous earth. Conventional methods for detecting microorganisms do not use the whole solution in at least 1 ml of the patient sample and can detect by extracting using only a portion of the nucleic acid. There is no big problem for large amounts of microorganisms, but for small amounts of microorganisms, it is not possible to accurately detect and control additional infectious diseases and there is a need for studies of concentration methods to utilize all microorganisms as much as possible in at least 1 ml of sample. Therefore, the present inventors have developed a technology capable of concentrating microorganisms about 4-8 times using the modified diatomaceous earth and directly extracting nucleic acids from the concentrated microorganism using dimethyl adipimidate (DMA). It is expected to significantly reduce external contamination, cost, time and complexity by enabling microbial concentration and nucleic acid extraction techniques in one tube.

### [Description of Drawings]

FIG. 1 shows FTIR spectra of diatomaceous earth before (blue) and after (red) modification with APTES.
FIG. 2 shows results of confirming experimental validation in the pathogen concentration process. D: diatomaceous earth, DA: diatomaceous earth modified with APTES.
FIG. 3 shows results of optimizing the pathogen concentration experimental conditions by changing the concentration of the modified diatomaceous earth.
FIG. 4 shows results of optimizing the pathogen concentration experimental conditions by varying the reaction time.
FIG. 5 shows results of optimizing the pathogen concentration experiment conditions by varying the concentration of *Brucella.*
FIG. 6 shows results of optimizing the virus concentration experiment conditions by using modified diatomaceous earth.
FIG. 7 shows results of confirming experimental validation in the DNA extraction process. D: diatomaceous earth, DA: diatomaceous earth modified with APTES.
FIG. 8 shows results of optimizing DNA extraction experiment conditions by varying the concentration of diatomaceous earth (DA) modified with APTES.
FIG. 9 shows results of optimizing the DNA extraction experiment conditions by varying the reaction time.
FIG. 10 shows results of optimizing DNA extraction experiment conditions by varying the concentration of HCT-116 cell.
FIG. 11 shows results of confirming experimental validation in the RNA extraction process. D: diatomaceous earth, DA: diatomaceous earth modified with APTES.
FIG. 12 shows results of optimizing RNA extraction experiment conditions by varying the concentration of diatomaceous earth (DA) modified with APTES.
FIG. 13 shows results of optimizing the RNA extraction experimental conditions by varying the reaction time.
FIG. 14 shows results of optimizing RNA extraction experiment conditions by varying the concentration of HCT-116 cell.
FIG. 15 shows results of Q-PCR after concentrating pathogens and extracting DNA in one tube using modified diatomaceous earth.

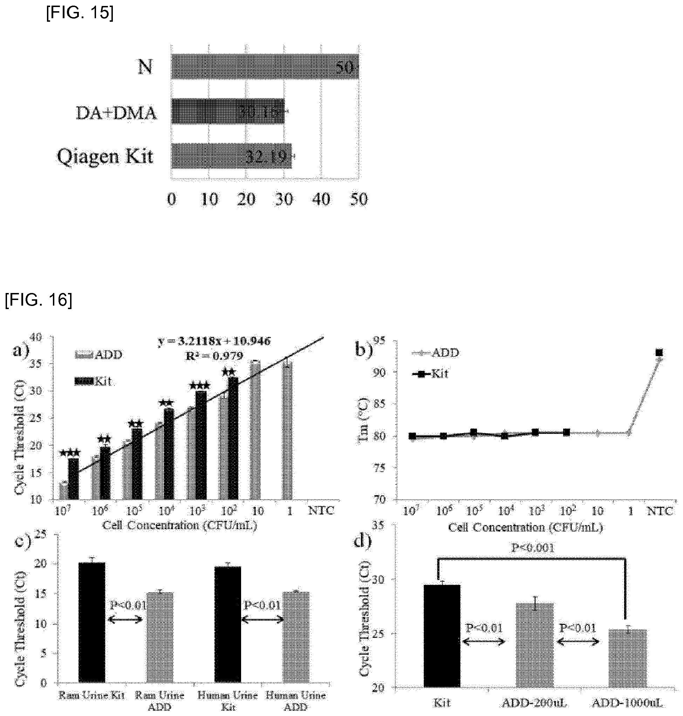

FIG. 16 shows experimental results of performance of the amine-functionalized diatomaceous earth-based, dimethyl suberimidate assisted system (ADD system) based on RNA extraction from pathogenic *Brucella* samples. (a) Ct values of RNA extracted from serially diluted cell concentrations of *Brucella* in PBS via both the improved DMS-assisted cross-linking procedures (gray) and traditional solid-phase extraction using a commercial kit (black). The linear relationship between the Ct value of RNA extracted by ADD and cell concentration is shown (R² = 0.979). (b) Tm values of extracted RNA determined by RT-qPCR. (c) Ct values of RNA extracted by both ADD and the kit for *Brucella* samples in urine at a concentration of 10⁷ CFU/mL. (d) Relatively large sample volume test of the ADD system for RNA extraction using HCT 116 cells at a concentration of 10³ cells/mL. The no-template control (NTC) showed no signal. Error bars indicate standard deviation from the mean of at least three independent experiments. The p-values were evaluated by Student t test (comparing both ADD and the kit with same concentration samples, ★★ p value <0.01, ★★★ p-value <0.001; p-value <0.01 indicates statistically significant, and p-value <0.001 indicates statistically highly significant).

### [Best Mode]

The present invention provides a composition for concentrating microorganisms comprising diatomaceous earth modified with a silane compound as an active ingredient.

Preferably, the silane compound may be a compound represented by the following Chemical Formula 1, but it is not limited thereto: 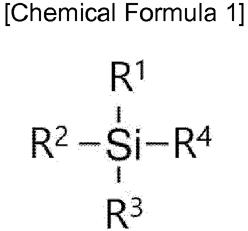 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

More preferably the silane compound may be any one or more selected from the group consisting of (3-aminopropyl)triethoxysilane (APTES), (3-aminopropyl)trimethoxysilane, (1-aminomethyl)triethoxysilane, (2-aminoethyl)triethoxysilane, (4-aminobutyl)triethoxysilane, (5-aminopentyl)triethoxysilane, (6-aminohexyl)triethoxysilane, 3-aminopropyl(diethoxy)methylsilane, N-[3-(trimethoxysilyl)propyl]ethylenediamine, [3-(2-aminoethylamino)propyl]trimethoxysilane (AEAPTMS) and 3-[(trimethoxysilyl)propyl]diethylenetriamine (TMPTA), but it is not limited thereto. More preferably, (3-aminopropyl)triethoxysilane (APTES) described in the example of the present invention is most suitable for the silane compound.

Preferably the microorganism may be any negatively charged microorganism such as bacteria, viruses or cells, more preferably *Brucella ovis* or para influenza virus (PIV3) described in the example of the present invention.

More preferably, the concentration of the microorganism may be 10⁰ cfu/ml to 10⁷ cfu/ml, but it is not limited thereto.

The present invention also provides a method of concentrating microorganisms comprising contacting a sample containing microorganism with diatomaceous earth modified with a silane compound.

Preferably, the silane compound may be a compound represented by the following Chemical Formula 1, but it is not limited thereto: 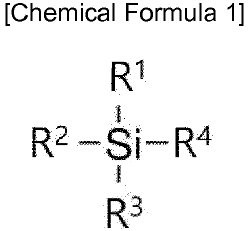 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

More preferably, (3-aminopropyl)triethoxysilane (APTES) described in the example of the present invention is most suitable for the silane compound.

Preferably, the sample containing microorganisms may be feces, urine, tears, saliva, external secretions of skin, external secretions of respiratory tract, external secretions of intestinal tract, external secretions of digestive tract, plasma, serum, blood, spinal fluid, lymph, body fluid and tissue, which belong to an object suspected of being infected with the microorganisms, but they are not limited thereto.

Preferably the microorganism may be any negatively charged microorganism such as bacteria, viruses or cells, more preferably *Brucella ovis* or para influenza virus (PIV3) described in the example of the present invention.

More preferably, the concentration of the microorganism may be 10⁰ cfu/ml to 10⁷ cfu/ml, but it is not limited thereto.

Preferably, the microorganism may be contacted with the diatomaceous earth modified with the silane compound for 20 to 60 minutes, but it is not limited thereto.

The present invention also provides a kit of concentrating microorganisms comprising the composition. The kit may further comprise a buffer solution and the like for adjusting pH required for effective microbial concentration.

The present invention also provides a composition for extracting nucleic acids comprising diatomaceous earth modified with a silane compound as an active ingredient.

Preferably, the silane compound may be a compound represented by the following Chemical Formula 1, but it is not limited thereto: 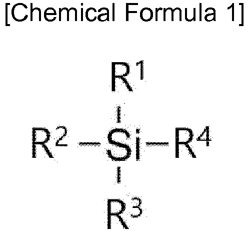 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

More preferably, (3-aminopropyl)triethoxysilane (APTES) described in the example of the present invention is most suitable for the silane compound.

Preferably, the composition may further any one or more selected from the group consisting of dimethyl adipimidate (DMA), dimethyl pimelimidate (DMP), dimethyl suberimidate (DMS) and dimethyl 3,3'-dithiobispropionimidate (DTBP).

Preferably, the nucleic acid may be DNA or RNA, but it is not limited thereto.

In addition, the present invention provides a method of extracting nucleic acids comprising: (1) reacting by adding diatomaceous earth modified with a silane compound to nucleic acid sample to be extracted; and (2) eluting nucleic acid from the reactant.

In addition, the present invention provides a method of extracting nucleic acids comprising: (1) mixing by adding diatomaceous earth modified with a silane compound to nucleic acid sample to be extracted; (2) reacting by adding any one or more selected from the group consisting of dimethyl adipimidate (DMA), dimethyl pimelimidate (DMP), dimethyl suberimidate (DMS) and dimethyl 3,3'-dithiobispropionimidate (DTBP); and (3) eluting nucleic acid from the reactant.

Preferably, the silane compound may be a compound represented by the following Chemical Formula 1, but it is not limited thereto: 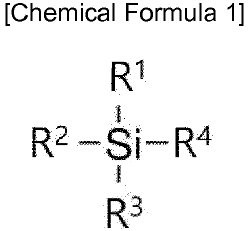 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

More preferably, (3-aminopropyl)triethoxysilane (APTES) described in the example of the present invention is most suitable for the silane compound.

Preferably, the nucleic acid may be DNA or RNA, but it is not limited thereto.

The present invention also provides a kit of extracting nucleic acids comprising the composition. The kit may further include a lysis buffer or a protease for lysing the cells in the sample to release the nucleic acid from inside the cell and may further comprise a buffer solution and the like for adjusting pH required for effective nucleic acid extraction.

The present invention also provides a composition for concentrating microorganisms and extracting nucleic acids comprising diatomaceous earth modified with a silane compound as an active ingredient.

Preferably, the silane compound may be a compound represented by the following Chemical Formula 1, but it is not limited thereto: 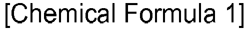 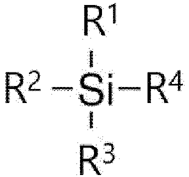 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

More preferably, (3-aminopropyl)triethoxysilane (APTES) described in the example of the present invention is most suitable for the silane compound.

Preferably the microorganism may be any negatively charged microorganism such as bacteria, viruses or cells, more preferably *Brucella ovis* or para influenza virus (PIV3) described in the example of the present invention.

More preferably, the concentration of the microorganism may be 10⁰ cfu/ml to 10⁷ cfu/ml, but it is not limited thereto.

Preferably, the composition may be prepared by further adding and reacting any one or more selected from the group consisting of dimethyl adipimidate (DMA), dimethyl pimelimidate (DMP), dimethyl suberimidate (DMS) and dimethyl 3,3'-dithiobispropionimidate (DTBP).

Preferably, the nucleic acid may be DNA or RNA, but it is not limited thereto.

In addition, the present invention provides a method of extracting nucleic acids from concentrated microorganism simultaneously with concentrating the microorganism comprising: (1) concentrating microorganism by contacting a sample containing microorganism with diatomaceous earth modified with a silane compound; (2) lysing the concentrated microorganism; and (3) eluting nucleic acid from the lysate.

In addition, the present invention provides a method of extracting nucleic acids from concentrated microorganism simultaneously with concentrating the microorganism comprising: (1) concentrating microorganism by contacting a sample containing microorganisms with diatomaceous earth modified with a silane compound; (2) lysing the concentrated microorganism; (3) reacting by adding any one or more selected from the group consisting dimethyl adipimidate (DMA), dimethyl pimelimidate (DMP), dimethyl suberimidate (DMS) and dimethyl 3,3'-ditihiobispropionimidate (DTBP) to the lysate; and (4) eluting nucleic acid from the reactant.

Preferably, the silane compound may be a compound represented by the following Chemical Formula 1, but it is not limited thereto. 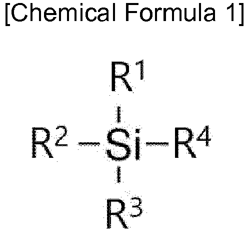 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

More preferably, (3-aminopropyl)triethoxysilane (APTES) described in the example of the present invention is most suitable for the silane compound.

Preferably, the sample containing microorganisms may be feces, urine, tears, saliva, external secretions of skin, external secretions of respiratory tract, external secretions of intestinal tract, external secretions of digestive tract, plasma, serum, blood, spinal fluid, lymph, body fluid and tissue, which belong to an object suspected of being infected with the microorganisms, but they are not limited thereto.

Preferably the microorganism may be any negatively charged microorganism such as bacteria, viruses or cells, more preferably *Brucella ovis* or para influenza virus (PIV3) described in the example of the present invention.

More preferably, the concentration of the microorganism may be 10⁰ cfu/ml to 10⁷ cfu/ml, but it is not limited thereto.

Preferably, the nucleic acid may be DNA or RNA, but it is not limited thereto.

The present invention also provides a kit of concentrating microorganisms and extracting nucleic acids comprising the composition. The kit may further include a lysis buffer or a protease for lysing the cells in the sample to release the nucleic acid from inside the cell and may further comprise a buffer solution and the like for adjusting pH required for effective microbial concentration and nucleic acid extraction.

Preferably, the kit may be in the form of a well-plate, a tube, a column or a microfluidic chip, but the kit may be in any commercially available form. It is possible. On the other hand, the well-plate may be manufactured without limitation, such as 96 wells, 384 wells.

The kit may be a single composition, or the individual components of the composition for concentrating microorganisms or extracting nucleic acids may be stored separately for mixing before its use. As such, the components of the composition for microbial concentration or nucleic acid extraction in the kit of the present invention may be packaged separately, or may be packaged in at least mixture in any combination of components. Each of the individual components or one or more mixture may be placed in different containers and the entire kit may be contained in a single container.

The present invention also provides the use of diatomaceous earth modified with a silane compound for microbial concentration.

The present invention also provides the use of diatomaceous earth modified with silane compounds for nucleic acid extraction.

The present invention also provides the use of diatomaceous earth modified with silane compounds for microbial concentration and nucleic acid extraction.

Hereinafter, the present invention will be described in detail with reference to the following examples. The examples are only for describing the present invention in more detail and it is obvious to those skilled in the art that that the scope of the present invention is not limited by these examples embodiments in accordance with the gist of the present invention.

### <Example 1> Modification of diatomaceous earth with 3-aminopropyltriethoxysilane (APTES)

For modifying APTES on diatomaceous earth, 1g of diatomaceous earth powder is washed by soaking in distilled water for 30 minutes and then washed in 70% EtOH and 99% EtOH sequentially for 30 minutes respectively and then completely dried using vacuum. The dried 500 mg of diatomaceous earth is added into 80 ml of 98% EtOH solution, and 2.4 ml of APTES is added and reacted for 4 hours at room temperature. After washing with the distilled water, its final concentration of 50mg/ml is prepared for using and storing.

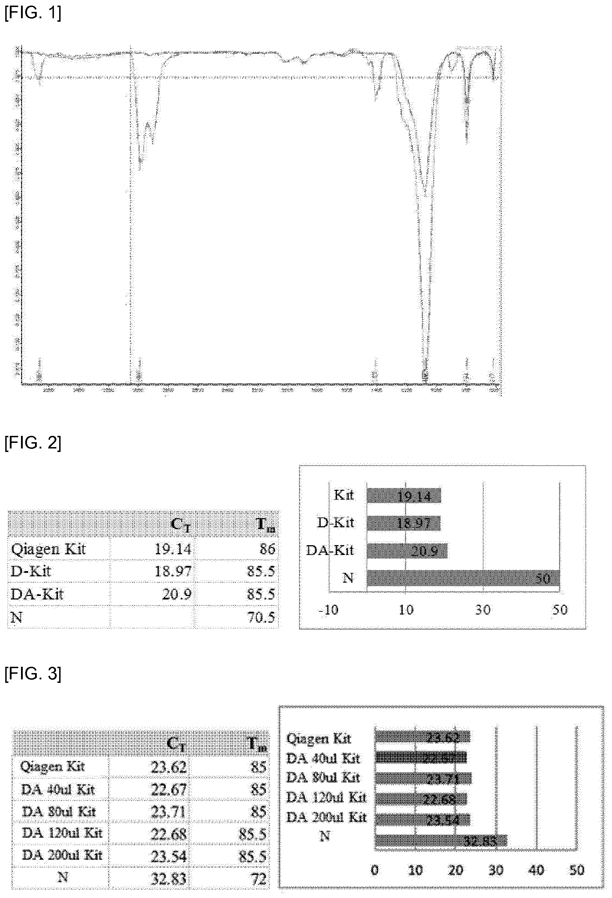

The FTIR spectra of diatomaceous earth before (blue) and after (red) modifying with APTES are shown in FIG. 1. After the modification with APTES, a strong peak appeared at 2987 nm, where the primary amine group is functionalized.

### <Example 2> Pathogen concentration process

*Brucella ovis* was used as the pathogen to be tested. For the experiment, 1 ml of 10⁶ cfu/ml *Brucella* solution was used.

### 1. Confirmation of experiment validation

1) 200 µl of sample was taken, DNA was extracted with Qiagen DNA Kit, and it was used as a control.
2) Each 1 ml of sample was concentrated using diatomaceous earth (D) and diatomaceous earth modified with APTES (DA). The concentration process is as follows.
3) 200 µl elution buffer was used to concentrate the pathogen. After centrifugation, DNA was extracted from the supernatant by Qiagen DNA Kit.
4) As shown in FIG. 2, the Q-PCR result confirmed that the diatomaceous earth can concentrate the pathogen.

### 2. Optimization of experimental conditions

Experiments were performed by varying the concentrations of the modified diatomaceous with 40 µl, 80 µl, 120 µl and 200 µl of 50 mg/ml modified diatomaceous earth (DA) solution. The balance was adjusted with the distilled water. For example, 160 µl of the distilled water was added to 40 µl of the modified diatomaceous earth (DA) solution. 1 ml of 10⁴ cfu/ml *Brucella* solution was used. As shown in FIG. 3, the Q-PCR results showed the optimum effect when using 40 µl of 50 mg/ml modified diatomaceous earth (DA) solution.

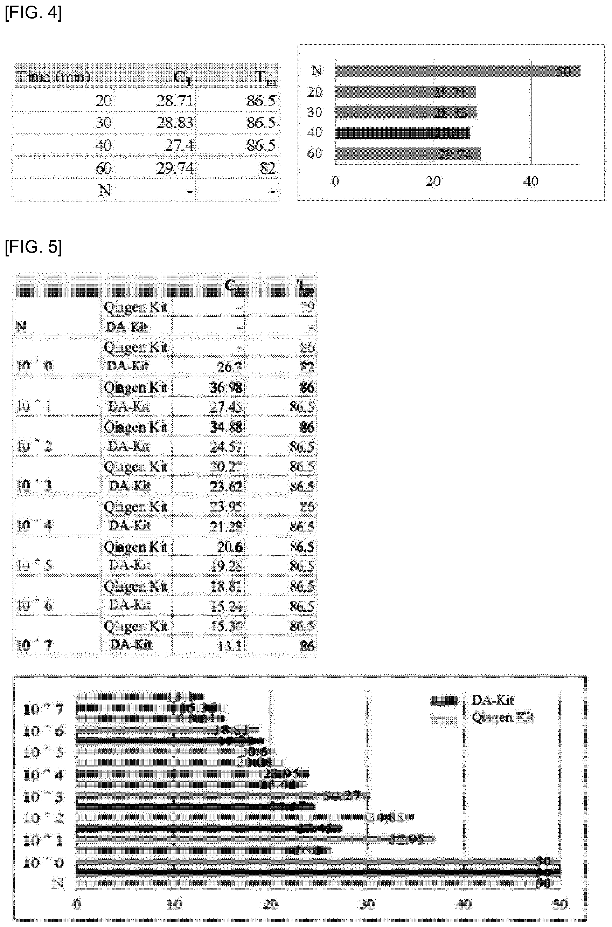

In addition, experiments were performed with different reaction times. The reaction time was varied from 20 to 60 minutes. 1 ml of 10³ cfu/ml *Brucella* solution was used. As shown in FIG. 4, the Q-PCR results showed an optimal effect at a reaction time of 40 minutes.

In addition, experiments were performed by varying the concentration of *Brucella.* The concentrations of *Brucella* were varied from 10⁰ cfu/ml to 10⁷ cfu/ml. As shown in FIG. 5, the Q-PCR results showed that the use of diatomaceous earth successfully concentrated the pathogens, and more effectively than the control group, Qiagen kit at low concentration.

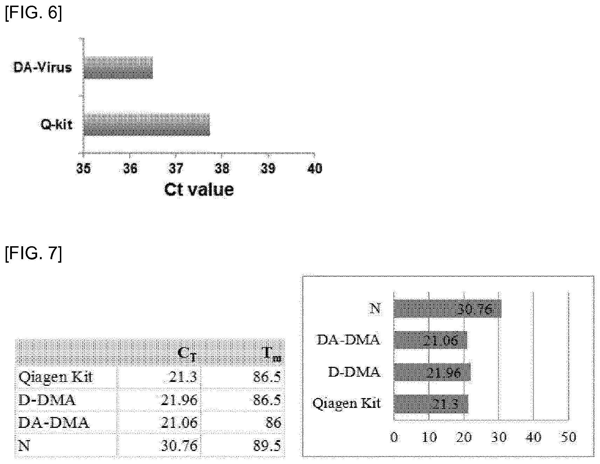

In addition, para-influenza virus (PIV3) concentration experiment was performed using the same conditions. When comparing the experiment using the conventional method (Qiagen kit) and the modified diatomaceous earth of 2 ml patient sample containing para-influenza virus (PIV3), of which the exact concentration does not known, it was confirmed that the modified diatomaceous earth was twice concentrated as the conventional method (FIG. 6).

### <Example 3> DNA extraction process

HCT-116 cancer cells were used as test samples. For the experiment, 1 ml of 10⁵ cells/ml cell solution was used. 100 µl of an elution buffer was used for final DNA extraction.

### 1. Confirmation of validation of DNA extraction experiment

1) 200 µl of sample was taken, DNA was extracted with Qiagen DNA Kit, and it was used as a control.
2) DNA was extracted using diatomaceous earth (D) and diatomaceous earth modified with APTES (DA), and dimethyl adipimidate (DMA) was added. The detailed extraction process is as follows:
   i. 200 µl of sample is transferred into a 2 ml microcentrifuge tube.
   ii. 200 µl of lysis buffer and 20 µl of Protease K are added to the sample and mixed 10 times with a pipette. Be careful not to create air bubbles at this time.
   iii. The sample is allowed to react at room temperature until the sample becomes clear (within 1 minute).
   iv. 80 µl of 50 mg/ml DA solution is added to 220 µl of the sample (sample of iii above) and mixed 5 times with a pipette.
   v. 100 µl of DMA is added to the mixture and mixed 10 times with a pipette carefully to avoid air bubbles.
   vi. The reaction is carried out at 56 °C for 20 minutes as to bind DNA to the modified diatomaceous earth (DA). The mixture is well dispersed during the reaction time.
   vii. The mixture is centrifuged for 1 minute using a microcentrifuge. The supernatant is removed carefully.
   viii. 100 µl of PBS buffer is added and mixed 10 times with a pipette.
   ix. The mixture is centrifuged for 1 minute. The supernatant is removed carefully to avoid air bubbles.
   x. It is rinsed twice.
   xi. 100 µl of elution buffer (pH 11) is added to the sample and mixed 15 times with a pipette.
   xii. The reaction is performed at room temperature (within 1 minute).
   xiii. The mixture is centrifuged for 1 minute. The supernatant was transferred by using a pipette to a new microtube.
3) As shown in FIG. 7, the Q-PCR result showed that diatomaceous earth modified with APTES (DA) was effective for DNA extraction.

### 2. Optimization of DNA extraction experiment conditions

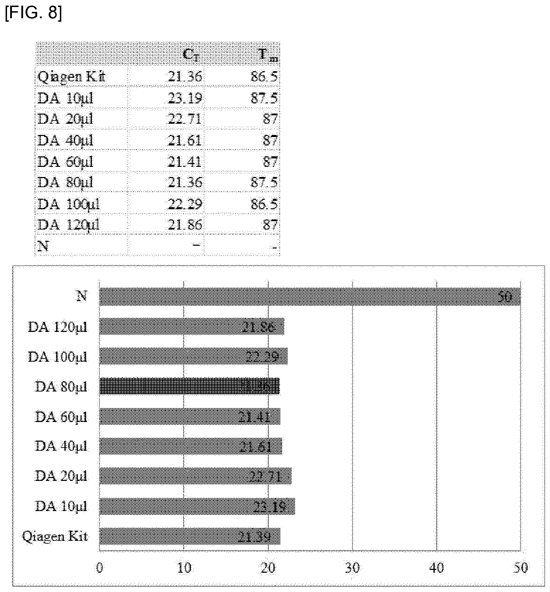

Experiments were performed with varying the concentration of diatomaceous earth modified with APTES (DA). 10-120 µL of 50 mg/ml DA solution was used. The balance was adjusted with the distilled water. For example, 160 µl of the distilled water was added to 40 µl of DA solution. 1 ml of 10⁵ cells/ml HCT-116 solution was used. As shown in FIG. 8, the Q-PCR results showed an optimal effect on DNA extraction when 40 µl of 50 mg/ml DA solution was used.

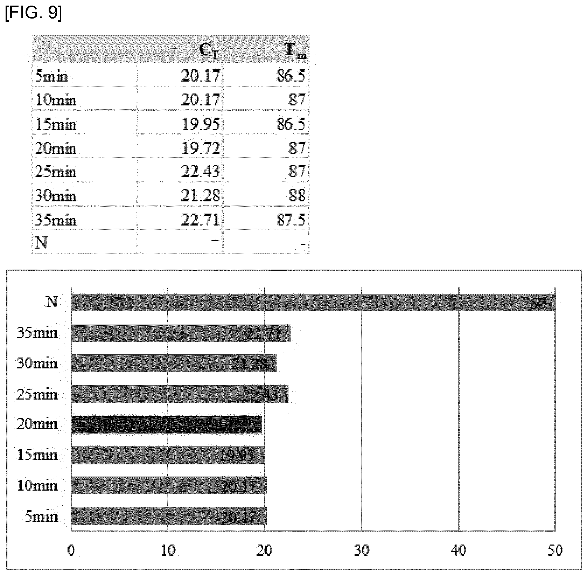

In addition, experiments were carried out with different reaction times. The reaction time was varied from 5 to 35 minutes. 1 ml of 10⁵ cells/ml HCT-116 solution was used. As shown in FIG. 9, the Q-PCR results showed an optimal effect on DNA extraction at a reaction time of 20 minutes.

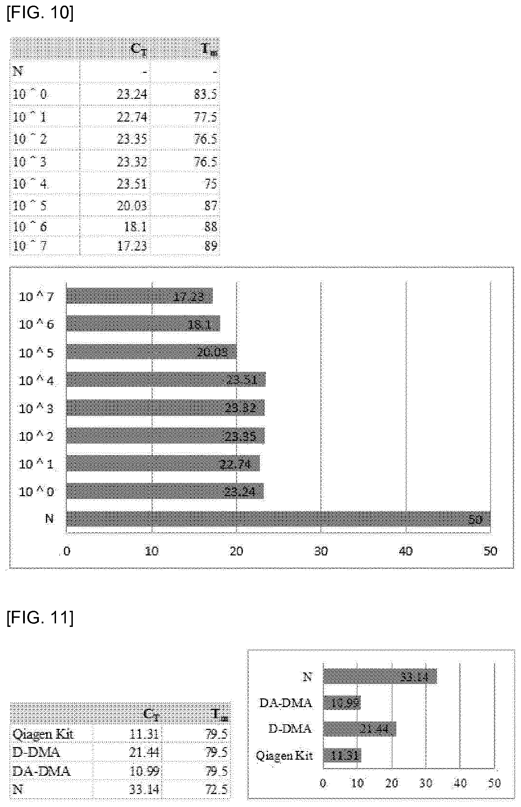

In addition, experiments were performed with varying the concentration of HCT-116 cell. The concentrations of HCT-116 cell were varied from 10⁰ cells/ml to 10⁷ cells/ml. As shown in FIG. 10, the Q-PCR results showed that DNA extraction was effective even at low cell concentrations.

### <Example 4> RNA extraction process

HCT-116 cancer cells were used as test samples. For the experiment, 1 ml of 10⁵ cells/ml cell solution was used. 100 µl elution buffer was used for final RNA extraction.

### 1. Validation of RNA Extraction Experiment

1) 200 µl of sample was taken, RNA was extracted with Qiagen DNA Kit, and it was used as a control.
2) RNA was extracted using diatomaceous earth (D) and diatomaceous earth (DA) modified with APTES, and dimethyl adipimidate (DMA) was added. It is similar to the above DNA extraction process except for the following three parts.
   i. 20 µl of DNase was treated and followed by the treatment with 20 µl of Protease K.
   ii. 40 µl of DA was used for RNA extraction.
   iii. The reaction was carried out with shaking for 25 minutes at room temperature.
3) As shown in FIG. 11, Q-PCR results showed that diatomaceous earth modified with APTES (DA) was effective for the RNA extraction.

### 2. Optimization of RNA extraction experiment conditions

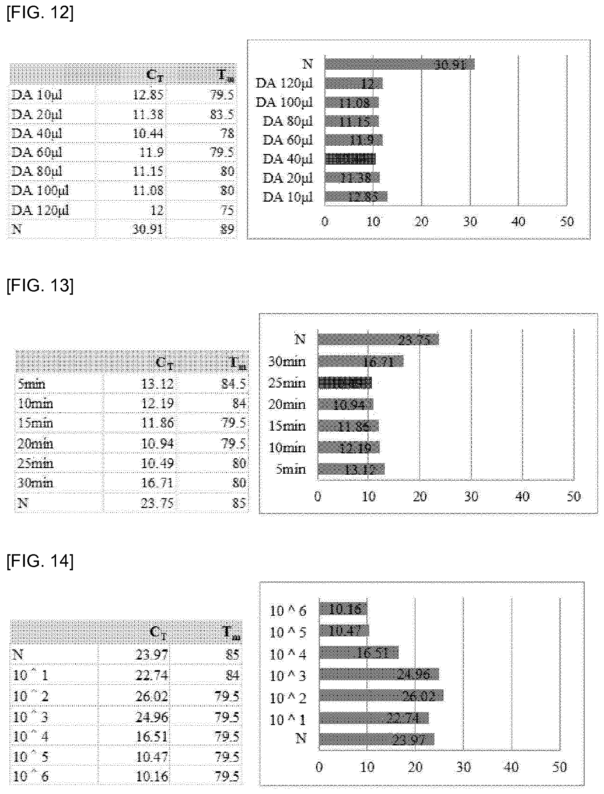

Experiments were performed with varying the concentrations of diatomaceous earth modified with APTES (DA). 10-120 µL of 50 mg/ml DA solution was used. The balance was adjusted with the distilled water. For example, 160 µl of the distilled water was added to 40 µl of the DA solution. 1 ml of 10⁵ cells/ml HCT-116 solution was used. As shown in FIG. 12, the Q-PCR results showed an optimal effect on RNA extraction when 40 µl of 50 mg/ml DA solution was used.

In addition, experiments were performed with varying reaction times. The reaction time was varied from 5 to 35 minutes. 1 ml of 10⁵ cells/ml HCT-116 solution was used. As shown in FIG. 13, the Q-PCR results showed an optimal effect on RNA extraction at a reaction time of 25 minutes.

In addition, experiments were performed with varying the concentration of HCT-116 cell. The concentrations of HCT-116 cell were varied from 10¹ cells/ml to 10⁶ cells/ml. As shown in FIG. 14, the Q-PCR results showed that RNA extraction was effective even at low cell concentrations.

### <Example 5> Pathogenic concentration and DNA Extraction process in one tube

Since diatomaceous earth can successfully concentrate pathogens and extract nucleic acids effectively, the present inventors designed an experiment capable of concentrating pathogens and extracting DNA from one tube without large equipment. *Brucella ovis* was used as the test pathogen. For the experiment, 1 ml of 10⁵ cfu/ml *Brucella* solution was used.
1) 100 µl of sample was taken, DNA was extracted with Qiagen DNA Kit, and it was used as a control.
2) 40 µl of 50 mg/ml diatomaceous earth solution modified with APTES (DA) was added to 1 ml of 10⁵ cfu/ml of *Brucella ovis* sample. The reaction was carried out with shaking for 40 minutes and well mixed to avoid precipitates.
3) After centrifugation, all supernatant was removed and washed twice with 1 ml distilled water.
5) 100 µl of elution buffer was added. After reacting for 1 minute, it was sunk slightly.
6) 100 µl of elution buffer containing 20 µl of Protease K was added to the mixture and mixed 10 times with a pipette.
7) 100 µl of 100 mg/ml DMA was added to the mixture and mixed 10 times with a pipette.
8) The reaction was carried out with shaking at 650 RPM for 20 minutes at 56 °C. and mixed well every 5 minutes.
9) All supernatant was removed by sinking and washed twice with 200 µl of PBS buffer (1X, pH 7.4).
10) 100 µl of elution buffer was added and reacted for 1 minute at room temperature.
11) The supernatant containing the extracted DNA was transferred to a 1.5 ml tube.

As shown in FIG. 15, it was shown that the pathogens can be concentrated and DNA can be extracted successfully in one tube, even without large equipment.

### <Example 6> Application of amine-modified diatomaceous earth-based, dimethyl suberimidate assisted system (ADD system) for diagnostics

The ADD system of the present invention can successfully extract RNAfrom biological samples. The optimized protocol was compared with a traditional approach, which showed that the ADD system may be useful for nucleic acid-based diagnostics. Human brucellosis, which is endemic in many countries and regions, is among the major zoonotic diseases worldwide. The present inventors used the ADD system to extract RNA from *Brucella* bacteria. No detectable signals from the No RT controls were present in any RT reactions. As shown in FIG. 16a, an ultralow *Brucella* concentration of 10⁰ CFU/mL was detected using the ADD system, which is 100-fold higher than the performance of the conventional kit. The relationship between Ct values of RNA extracted by ADD and pathogen cell concentration presented a highly linear correlation (R² = 0.979). Fluorescence signals were confirmed to be specific based on the Tm and no-template control (NTC) (in FIG. 16b). Additionally, RNA extracted from a 10⁵ CFU/mL *Brucella* sample from urine was further evaluated using the ADD system of the present invention to determine the clinical relevance thereof. Because *Brucella* is a zoonotic pathogen, the present inventors spiked the same concentration of *Brucella* into both human urine and ovine urine samples. Although the overall Ct value of *Brucella* in urine was delayed compared to that in PBS, the quality of RNA extracted using the ADD system showed an earlier Ct result compared to RNA extracted using the kit (FIG. 16c).

In addition to *Brucella,* the present inventors also successfully extracted RNA from *Salmonella* and *E. coli* pathogens following the same optimized protocol. All RNA samples extracted by ADD presented earlier Ct values than those of the conventional kit. The present inventors also tested our ADD system with relatively large sample volumes to increase the detection limit by loading more samples. The RT-qPCR results showed that more RNA was extracted from a large-volume (1 mL) sample by the ADD system than by using other systems (FIG. 16d). The Ct value for RNA from the 1 mL sample was approximately 2 cycles earlier than that of the 200 µL sample, indicating that the amount of extracted RNA was clearly increased when the sample volume was larger. For RNA extracted using the ADD system, although the protocol was optimized for 200 µL samples, larger volumes also can be used. Therefore, the ADD system proposed by the present invention is useful for RNA extraction and detection of pathogens based on nucleic acid amplification by RT-qPCR.

## Claims

1. A composition for concentrating microorganisms comprising diatomaceous earth modified with a silane compound as an active ingredient.

2. The composition for concentrating microorganisms of claim 1, wherein the silane compound is a compound represented by a following Chemical Formula 1: 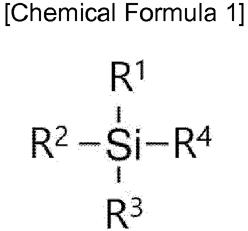 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

3. The composition for concentrating microorganisms of claim 2, wherein the silane compound is any one or more selected from the group consisting of (3-aminopropyl)triethoxysilane (APTES), (3-aminopropyl)trimethoxysilane, (1-aminomethyl)triethoxysilane, (2-aminoethyl)triethoxysilane, (4-aminobutyl)triethoxysilane, (5-aminopentyl)triethoxysilane, (6-aminohexyl)triethoxysilane, 3-aminopropyl(diethoxy)methylsilane, N-[3-(trimethoxysilyl)propyl]ethylenediamine, [3-(2-aminoethylamino)propyl]trimethoxysilane (AEAPTMS) and 3-[(trimethoxysilyl)propyl]diethylenetriamine (TMPTA).

4. The composition for concentrating microorganisms of claim 1, wherein the microorganism is bacterium, virus or cell.

5. A method of concentrating microorganisms comprising contacting a sample containing microorganisms with diatomaceous earth modified with a silane compound.

6. The method of concentrating microorganisms of claim 5, wherein the sample containing microorganisms is any one selected from the group consisting of feces, urine, tears, saliva, external secretions of skin, external secretions of respiratory tract, external secretions of intestinal tract, external secretions of digestive tract, plasma, serum, blood, spinal fluid, lymph, body fluid and tissue, which belong to an object suspected of being infected with the microorganisms.

7. The method of concentrating microorganisms of claim 5, wherein the microorganism is bacterium, virus or cell.

8. A kit of concentrating microorganisms comprising the composition of any one of claims 1 to 4.

9. A composition for extracting nucleic acids comprising diatomaceous earth modified with a silane compound as an active ingredient.

10. The composition for extracting nucleic acids of claim 9, wherein the silane compound is a compound represented by a following Chemical Formula 1: 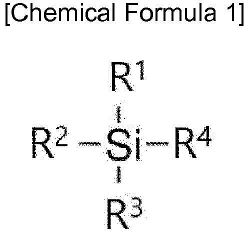 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

11. The composition for extracting nucleic acids of claim 9, further comprising any one or more selected from the group consisting of dimethyl adipimidate (DMA), dimethyl pimelimidate (DMP), dimethyl suberimidate (DMS) and dimethyl 3,3'-dithiobispropionimidate (DTBP).

12. The composition for extracting nucleic acids of claim 9, wherein the nucleic acid is DNA or RNA.

13. A method of extracting nucleic acids comprising:
(1) reacting by adding diatomaceous earth modified with a silane compound to nucleic acid sample to be extracted; and
(2) eluting nucleic acid from a reactant.

14. A method of extracting nucleic acids comprising:
(1) mixing by adding diatomaceous earth modified with a silane compound to nucleic acid sample to be extracted;
(2) reacting by adding any one or more selected from the group consisting of dimethyl adipimidate (DMA), dimethyl pimelimidate (DMP), dimethyl suberimidate (DMS) and dimethyl 3,3'-dithiobispropionimidate (DTBP); and
(3) eluting nucleic acid from a reactant.

15. A kit of extracting nucleic acids comprising the composition of any one of claims 9 to 12.

16. A composition for concentrating microorganisms and extracting nucleic acids comprising diatomaceous earth modified with a silane compound as an active ingredient.

17. The composition for concentrating microorganisms and extracting nucleic acids of claim 16, wherein the silane compound is a compound represented by a following Chemical Formula 1: 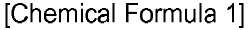 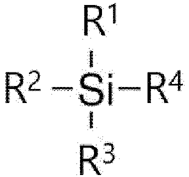 wherein R¹ to R³ may each be the same or different and any one of C1 to C4 alkyl or C1 to C4 alkoxy, and R⁴ is any one of amino(C1 to C10)alkyl, 3-(2-amino(C1 to C4)alkylamino) (C1 to C4) alkyl or 3-[2-(2-amino(C1 to C4)alkylamino) (C1 to C4) alkylamino] (C1 to C4) alkyl.

18. The composition for concentrating microorganisms and extracting nucleic acids of claim 16, further comprising any one or more selected from the group consisting of dimethyl adipimidate (DMA), dimethyl pimelimidate (DMP), dimethyl suberimidate (DMS) and dimethyl 3,3'-dithiobispropionimidate (DTBP).

19. The composition for concentrating microorganisms and extracting nucleic acids of claim 16, wherein the nucleic acid is DNA or RNA.

20. A method of extracting nucleic acids from concentrated microorganism simultaneously with concentrating the microorganism comprising:
(1) concentrating microorganism by contacting a sample containing microorganism with diatomaceous earth modified with a silane compound;
(2) lysing concentrated microorganism; and
(3) eluting nucleic acid from lysate.

21. A method of extracting nucleic acids from concentrated microorganism simultaneously with concentrating the microorganism comprising:
(1) concentrating microorganism by contacting a sample containing microorganisms with diatomaceous earth modified with a silane compound;
(2) lysing concentrated microorganism;
(3) reacting by adding any one or more selected from the group consisting dimethyl adipimidate (DMA), dimethyl pimelimidate (DMP), dimethyl suberimidate (DMS) and dimethyl 3,3'-ditihiobispropionimidate (DTBP) to lysate; and
(4) eluting nucleic acid from a reactant.

22. A kit for concentrating microorganisms and extracting nucleic acids comprising the composition of any one of claims 16 to 19.

23. The kit for concentrating microorganisms and extracting nucleic acids of claim 22, wherein the kit is any one type selected from the group consisting of a well-plate, a tube, a column and a microfluidic chip.
